(19) Europäisches Patentamt
European Patent Office
Office européen des brevets

(11) EP 2 157 858 B1

(12) **EUROPEAN PATENT SPECIFICATION**

(45) Date of publication and mention of the grant of the patent:
**22.08.2012 Bulletin 2012/34**

(21) Application number: **08760926.9**

(22) Date of filing: **12.06.2008**

(51) Int Cl.:
***A01N 43/12*** *(2006.01)* ***A01N 43/08*** *(2006.01)*
***A01N 43/653*** *(2006.01)* ***A01N 43/56*** *(2006.01)*
***A01N 37/50*** *(2006.01)* ***A01N 37/46*** *(2006.01)*
***A01P 7/04*** *(2006.01)*

(86) International application number:
**PCT/EP2008/057385**

(87) International publication number:
**WO 2008/152092 (18.12.2008 Gazette 2008/51)**

(54) **PESTICIDAL COMPOSITION COMPRISING A STRIGOLACTONE DERIVATIVE AND AN FUNGICIDE COMPOUND**

PESTIZIDZUSAMMENSETZUNG MIT EINEM STRIGOLACTONDERIVAT UND EINER FUNGIZIDVERBINDUNG

COMPOSITION PESTICIDE COMPRENANT UN DERIVE DE STRIGOLACTONE ET UN COMPOSE FONGICIDE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.06.2007 EP 07356084**

(43) Date of publication of application:
**03.03.2010 Bulletin 2010/09**

(73) Proprietor: **Bayer CropScience AG**
**40789 Monheim (DE)**

(72) Inventors:
- **SUTY-HEINZE, Anne**
**40764 Langenfeld (DE)**
- **VORS, Jean-Pierre**
**69110 Sainte Foy Les Lyon (FR)**

(74) Representative: **Guitton, Carole et al**
**Bayer CropScience SA**
**Patents & Licensing Department**
**14-20 rue Pierre Baizet - BP9163**
**69263 Lyon cedex 09 (FR)**

(56) References cited:
**WO-A-2005/077177**

- **JUAN C.G. GALINDO ET AL.: "SAR studies of sesquiterpene lactones as Orobanche cumana seed germination stimulants" JOURNAL OF AGRICULTURAL AND FOOD CHEMISTRY, vol. 50, 2002, pages 1911-1917, XP007903570**

Remarks:
The file contains technical information submitted after the application was filed and not included in this specification

EP 2 157 858 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention relates to pesticidal compositions comprising a strigolactone derivative and a fungicide compound. The present invention also relates to a method of combating or controlling pests and diseases or to improve yield, growth or vigor of a plant by applying at a locus such a composition.

Strigolactones have been identified in the root exudates of a variety of plant species, and have been initially disclosed as compounds capable of stimulating the seed germination of parasitic weed species, especially *Orobanche sp.* and *striga sp.* Several strigolactones have been identified, including strigol, sorgolactone, alectrol, orobanchol (Cook et al., 1972, J Am Chem Soc, 94, 6198-6199; Butler, 1995, Allelopathy: organism, processes and application, 158-168; Hauck et al., 1992, J Plant Physiol, 139, 474-478; Muller et al., 1992, J Plant Growth Regul, 11, 77-84, Siame et al., 1993, J Agr Food Chem, 41, 1486- 1491, Yokota et al., 1998, Phytochemistry, 49, 1967-1973).

More recently, it has been shown that strigolactones can stimulate the growth of arbuscular mycorrhizae (AM) fungi (WO 2005/077177). AM fungi are obligate symbionts incapable of completing their life cycle in the absence of a host root. The fungi penetrate and colonize plant roots, where they differentiate into highly branched structures known as arbuscules. More than 80% of lands plants are forming symbiotic associations with AM fungi.

The chemical structures of these natural strigolactones are represented in formula 1. They generally possess 4 cycles A, B, C, D and share the common configuration indicated below, in which cycles A and B can comprises double bond(s) or substituents:

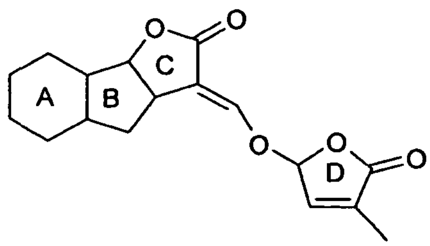

Additionally to these natural occurring compounds, a number of structural analogues have been designated, synthesized and biologically tested on the seed germination or as inducer of hyphal branching.

The connection of the C and D cycles to each other via an enol ether bond seems advantageous for germination stimulation. This C-D configuration is also advantageous for the effect of strigolactones on AM fungi, when modifications in the A and B cycles do not appear to affect their ability to induce hyphal branching in AM fungi (Akiyama K. and H. Hayashi H., 2006, Annals of Botany, 97: 925-931).

These synthetic compounds, which may be identical to natural strigolactones or derivatives from them, are usually easier to be prepared in large amount than natural compounds extracted from root exsudates. They can therefore overcome a major drawback of the availability of natural compounds and are more suitable for a large scale application.

It is always of high-interest in agriculture to use novel pesticidal mixtures showing a broader scope of activity, or a fungicide or insecticide synergistic effect or a synergistic improvement in the plant stand, growth, and yield properties of the plants.

The composition according to the present invention may provide a synergistic effect. This synergistic effect allows a reduction of the chemical substances spread into the environment and a reduction of the cost of the treatment.

In the context of the present invention, the term "synergistic effect" is defined by Colby according to the article entitled "Calculation of the synergistic and antagonistic responses of herbicide combinations" Weeds, (1967), 15, pages 20-22.

The latter article mentions the formula:

$$E = x + y - \frac{x * y}{100}$$

in which E represents the expected percentage of inhibition of the disease for the combination of the two compounds at defined doses (for example equal to x and y respectively), x is the percentage of inhibition observed for the disease by the compound (I) at a defined dose (equal to x), y is the percentage of inhibition observed for the disease by the compound (II) at a defined dose (equal to y). When the percentage of inhibition observed for the combination is greater than E, there is a synergistic effect.

Such calculation can be done with the percentage of enhancement of plant characteristics such as plant stand, growth, vigor or yield in comparison to non-treated soil, seeds, seedlings, roots or plants, wherein an enhancement refers to an

improvement (which may be statistically significant) in any of the above plant characteristics in comparison to non-treated soil, seeds, seedlings, roots or plants.

The pesticidal compositions according to the invention have demonstrated significant improvement in the combination over the individual treatments alone with respect to plant growth, vigor or yield of plants or crops, or fungicide effect.

Additionally, the use of the compositions according to the invention in pre-treatment may also show a significant improvement in term of pesticidal efficacy due to the potential action of the strigolactones as suicidal germination agents. When used in pre-treatment, the compositions according to the invention can induce germination of the parasitic weed species, when their obligatory host is not present. The parasitic weeds therefore would die because they fail to find a host attachment.

Accordingly, the present invention provides a composition comprising:

**a) a strigolactone derivative of formula (I):**

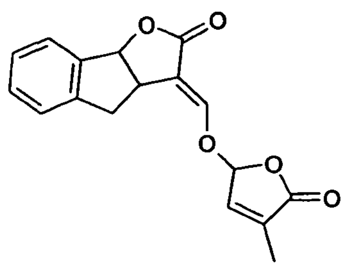

**(I)**

**and**

**b) a fungicide compound selected in the list L consisting of N-[2-(1,3-dimethylbutyl)pheny]-5-fluoro-1,3-dimethyl-1H-pyrazole-4-carboxamide, metalaxyl, metalaxyl-M, fluoxastrobin, trifloxystrobin, prothioconazole, tebuconazole, triadimenol;**

**in a (a)/(b) weight ratio of from 1/1 to 1/$10^{14}$**

Any of the compounds according to the present invention may also exist in one or more geometric isomeric form depending on the number of double bond within the compound. The invention thus equally relates to any geometric isomer and to any possible mixtures thereof, in any proportion. Geometric isomers can be separated according to any method known per se by the man ordinary skilled in the art.

According to the invention, the following generic terms are generally used with the following meanings:

- halogen means fluorine, chlorine, bromine or iodine;
- heteroatom can be nitrogen, oxygen or sulphur;

unless indicated otherwise, a group or a substituent that is substituted according to the invention can be substituted by one or more of the following groups or atoms: a halogen atom, a nitro group, a hydroxy group, a cyano group, an amino group, a sulphenyl group, a formyl group, a formyloxy group, a formylamino group, a carbamoyl group, a N-hydroxycarbamoyl group, a carbamate group, substituted or non-substituted (hydroxyimino)-$C_1$-$C_6$-alkyl group, substituted or non-substituted $C_1$-$C_8$-alkyl, substituted or non-substituted tri($C_1$-$C_8$-alkyl)silyl-$C_1$-$C_8$-alkyl, substituted or non-substituted $C_1$-$C_8$-cycloalkyl, substituted or non-substituted tri($C_1$-$C_8$-alkyl)silyl-$C_1$-$C_8$-cycloalkyl, substituted or non-substituted $C_1$-$C_8$-halogenoalkyl having 1 to 5 halogen atoms, substituted or non-substituted $C_1$-$C_8$-halogenocycloalkyl having 1 to 5 halogen atoms a $C_2$-$C_8$-alkenyl, substituted or non-substituted $C_2$-$C_8$-alkynyl, substituted or non-substituted $C_1$-$C_8$-alkylamino, substituted or non-substituted di-$C_1$-$C_8$-alkylamino, substituted or non-substituted $C_1$-$C_8$-alkoxy, substituted or non-substituted $C_1$-$C_8$-halogenoalkoxy having 1 to 5 halogen atoms, substituted or non-substituted $C_2$-$C_8$-alkenyloxy, substituted or non-substituted $C_2$-$C_8$-alkynyloxy, substituted or non-substituted $C_1$-$C_8$-alkylsulphenyl, substituted or non-substituted $C_1$-$C_8$-halogenoalkylsulphenyl having 1 to 5 halogen atoms, substituted or non-substituted $C_2$-$C_8$-alkenyloxy, substituted or non-substituted $C_2$-$C_8$-halogenoalkenyloxy having 1 to 5 halogen atoms, substituted or non-substituted $C_3$-$C_8$-alkynyloxy, substituted or non-substituted $C_3$-$C_8$-halogenoalkynyloxy having 1 to 5 halogen atoms, substituted or non-substituted $C_1$-$C_8$-alkylcarbonyl, substituted or non-substituted $C_1$-$C_8$-halogenoalkylcarbonyl having 1 to 5 halogen atoms, substituted or non-substituted $C_1$-$C_8$-alkylcarbamoyl, substituted or non-substituted di-$C_1$-$C_8$-alkylcarbamoyl, substituted or non-substituted N-C1-$C_8$-alkyloxycarbamoyl, substituted or non-substituted $C_1$-$C_8$-alkoxycarbamoyl, substituted or non-substituted N-$C_1$-$C_8$-alkyl-$C_1$-$C_8$-alkoxycarbamoyl, substituted or non-substituted $C_1$-$C_8$-

alkoxycarbonyl, substituted or non-substituted $C_1$-$C_8$-halogenoalkoxycarbonyl having 1 to 5 halogen atoms, substituted or non-substituted $C_1$-$C_8$-alkylcarbonyloxy, substituted or non-substituted $C_1$-$C_8$-halogenoalkylcarbonyloxy having 1 to 5 halogen atoms, substituted or non-substituted $C_1$-$C_8$-alkylcarbonylamino, substituted or non-substituted $C_1$-$C_8$-halogenoalkylcarbonylamino having 1 to 5 halogen atoms, substituted or non-substituted $C_1$-$C_8$-alkylaminocarbonyloxy, substituted or non-substituted di-$C_1$-$C_8$-alkylaminocarbonyloxy, substituted or non-substituted $C_1$-$C_8$-alkyloxycarbonyloxy, substituted or non-substituted $C_1$-$C_8$-alkylsulphenyl, substituted or non-substituted $C_1$-$C_8$-halogenoalkylsulphenyl having 1 to 5 halogen atoms, substituted or non-substituted $C_1$-$C_8$-alkylsulphinyl, substituted or non-substituted $C_1$-$C_8$-halogenoalkylsulphinyl having 1 to 5 halogen atoms, substituted or non-substituted $C_1$-$C_8$-alkylsulphonyl, substituted or non-substituted $C_1$-$C_8$-halogenoalkylsulphonyl having 1 to 5 halogen atoms, substituted or non-substituted $C_1$-$C_8$-alkylaminosulfamoyl, substituted or non-substituted di-$C_1$-$C_8$-alkylaminosulfamoyl, substituted or non-substituted ($C_1$-$C_6$-alkoxyimino)-$C_1$-$C_6$-alkyl, substituted or non-substituted ($C_1$-$C_6$-alkenyloxyimino)-$C_1$-$C_6$-alkyl, substituted or non-substituted ($C_1$-$C_6$-alkynyloxyimino)-$C_1$-$C_6$-alkyl, substituted or non-substituted (benzyloxyimino)-$C_1$-$C_6$-alkyl, substituted or non-substituted $C_1$-$C_8$-alkoxyalkyl, substituted or non-substituted $C_1$-$C_8$-halogenoalkoxyalkyl having 1 to 5 halogen atoms, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, a substituted or non-substituted or a 4-, 5-, 6- or 7-membered heterocycle comprising up to 4 heteroatoms selected in the list consisting of N, O, S.

The composition according to the present invention comprises (a) a compound of formula (I) and (b) a fungicide compound in a (a) / (b) weight ratio of from 1/1 to $1/10^{14}$. Preferably, (a) /(b) weight ratio is of from 1/10 to $1/10^{13}$. Even more preferably, (a) / (b) weight ratio is of from $1/10^2$ to $1/10^{12}$, from $1/10^2$ to $1/10^8$ from $1/10^3$ to $1/10^6$, and from $1/10^3$ to $1/10^5$.

When the composition is applied via a seed treatment, the (a)/(b) ratio can be advantageously from $1/10^2$ to $1/10^8$ , preferably from $1/10^3$ to $1/10^6$ , even more preferably from $1/10^3$ to $1/10^5$.

A man of ordinary skill in the art would be able to determine the adequate ratios according to the methods of application and to the compounds.

The composition of the present invention may further comprise at least one other different fungicide active ingredient (c).

Examples of suitable fungicide mixing partners may be selected in the following lists:

c1) a compound capable to inhibit the nucleic acid synthesis like benalaxyl, benalaxyl-M, bupirimate, clozylacon, dimethirimol, ethirimol, furalaxyl, hymexazol, mefenoxam, metalaxyl, metalaxyl-M, ofurace, oxadixyl, oxolinic acid ;
c2) a compound capable to inhibit the mitosis and cell division like benomyl, carbendazim, diethofencarb, ethaboxam, fuberidazole, pencycuron, thiabendazole, thiophanate-methyl, zoxamide ;
c3) a compound capable to inhibit the respiration for example
as CI-respiration inhibitor like diflumetorim ;
as CII-respiration inhibitor like boscalid, carboxin, fenfuram, flutolanil, furametpyr, furnecyclox, mepronil, oxycarboxin, penthiopyrad, thifluzamide ;
as CIII-respiration inhibitor like amisulbrom, azoxystrobin, cyazofamid, dimoxystrobin, enestrobin, famoxadone, fenamidone, fluoxastrobin, kresoxim-methyl, metominostrobin, orysastrobin, picoxystrobin, pyraclostrobin, trifloxystrobin ;
c4) a compound capable of to act as an uncoupler like dinocap, fluazinam, meptyldinocap;
c5) a compound capable to inhibit ATP production like fentin acetate, fentin chloride, fentin hydroxide, silthiofam;
c6) a compound capable to inhibit AA and protein biosynthesis like andoprim, blasticidin-S, cyprodinil, kasugamycin, kasugamycin hydrochloride hydrate, mepanipyrim, pyrimethanil;
c7) a compound capable to inhibit the signal transduction like fenpiclonil, fludioxonil, quinoxyfen;
c8) a compound capable to inhibit lipid and membrane synthesis like biphenyl, chlozolinate, edifenphos, etridiazole, iodocarb, iprobenfos, iprodione, isoprothiolane, procymidone, propamocarb, propamocarb hydrochloride, pyrazophos, tolclofos-methyl, vinclozolin ;
c9) a compound capable to inhibit ergosterol biosynthesis like aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, prochloraz, propiconazole, prothioconazole, pyributicarb, pyrifenox, simeconazole, spiroxamine, tebuconazole, terbinafine, tetraconazole, triadimefon, triadimenol, tridemorph, triflumizole, triforine, triticonazole, uniconazole, viniconazole, voriconazole ;
c10) a compound capable to inhibit cell wall synthesis like benthiavalicarb, dimethomorph, flumorph, iprovalicarb, mandipropamid, polyoxins, polyoxorim, validamycin A;

c11) a compound capable to inhibit melanine biosynthesis like carpropamid, diclocymet, fenoxanil, phthalide, pyroquilon, tricyclazole;

c12) a compound capable to induce a host defence like acibenzolar-S-methyl, probenazole, tiadinil;

c13) a compound capable to have a multisite action like Bordeaux mixture, captafol, captan, chlorothalonil, copper naphthenate, copper oxide, copper oxychloride, copper preparations such as copper hydroxide, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propineb, sulphur and sulphur preparations including calcium polysulphide, thiram, tolylfluanid, zineb, ziram ;

c14) a compound selected in the following list: (2E)-2-(2-{[6-(3-chloro-2-methylphenoxy)-5-fluoropyrimidin-4-yl]oxy}phenyl)-2-(methoxyimino)-N-methylacetamide, (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, 1-(4-chlorophenyl)-2-(1H-1,2,4-triazol-1-yl)cycloheptanol, 1-[(4-methoxyphenoxy)methyl]-2,2-dimethylpropyl-1H-imidazole-1-carboxylate, 1-methyl-N-[2- (1,1,2,2-tetrafluoroethoxy)phenyl]-3-(trifluoromethyl)-1H-pyrazole-4-carboxamide, 2,3,5,6-tetrachloro-4-(methylsulfonyl)pyridine, 2-butoxy-6-iodo-3-propyl-4H-chromen-4-one, 2-chloro-N-(1,1,3-trimethyl-2,3-dihydro-1H-inden-4-yl)nicotinamide, 2-phenylphenol and salts, 3-(difluoromethyl)-1-methyl-N-[2-(1,1,2,2-tetrafluoroethoxy)phenyl]-1H-pyrazole-4-carboxamide, 3-(difluoromethyl)-N-[(9R)-9-isopropyl-1,2,3,4-tetrahydro-1,4-methanonaphthalen-5-yl]-1-methyl-1H-pyrazole-4-carboxamide, 3-(difluoromethyl)-N-[(9S)-9-isopropyl-1,2,3,4-tetrahydro-1,4-methanonaphthalen-5-yl]-1-methyl-1H-pyrazole-4-carboxamide, 3-(difluoromethyl)-N-[4'-(3,3-dimethylbut-1-yn-1-yl)biphenyl-2-yl]-1-methyl-1H-pyrazole-4-carboxamide, 3,4,5-trichloropyridine-2,6-dicarbonitrile, 3-[5-(4-chlorophenyl)-2,3-dimethylisoxazolidin-3-yl]pyridine, 3-chloro-5-(4-chlorophenyl)-4-(2,6-difluorophenyl)-6-methylpyridazine , 4-(4-chlorophenyl)-5-(2,6-difluorophenyl)-3,6-dimethylpyridazine, 5-chloro-7-(4-methylpiperidin-1-yl)-6-(2,4,6-trifluorophenyl)[1,2,4]triazolo[1,5-a]pyrimidine, 8-hydroxyquinoline sulfate, benthiazole, bethoxazin, capsimycin, carvone, chinomethionat, cufraneb, cyflufenamid, cymoxanil, dazomet, debacarb, dichlorophen, diclomezine, dicloran, difenzoquat, difenzoquat methylsulphate, diphenylamine, ecomate, ferimzone, flumetover, fluopicolide, fluoroimide, flusulfamide, fosetyl-aluminium, fosetyl-calcium, fosetyl-sodium, hexachlorobenzene, irumamycin, isotianil, methasulfocarb, methyl (2E)-2-{2-[({cyclopropyl[(4-methoxyphenyl)imino]methyl}thio)methyl]phenyl}-3-methoxyacrylate, methyl 1-(2,2-dimethyl-2,3-dihydro-1H-inden-1-yl)-1H-imidazole-5-carboxylate, methyl isothiocyanate, metrafenone, mildiomycin, N-(3',4'-dichloro-5-fluorobiphenyl-2-yl)-3-(difluoromethyl)-1-methyl-1H-pyrazole-4-carboxamide, N-(3-ethyl-3,5,5-trimethylcyclohexyl)-3-(formylamino)-2-hydroxybenzamide, N-(4-chloro-2-nitrophenyl)-N-ethyl-4-methylbenzenesulfonamide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloronicotinamide, N-[1-(5-bromo-3-chloropyridin-2-yl)ethyl]-2,4-dichloronicotinamide, N-[1-(5-bromo-3-chloropyridin-2-yl)ethyl]-2-fluoro-4-iodonicotinamide, N-[2-(1,3-dimethylbutyl)phenyl]-5-fluoro-1,3-dimethyl-1H-pyrazole-4-carboxamide, N-{(Z)-[(cyclopropylmethoxy)imino][6-(difluoromethoxy)-2,3-difluorophenyl]methyl}-2-phenylacetamide, N-{2-[1,1'-bi(cyclopropyl)-2-yl]phenyl} -3-(difluoromethyl)-1-methyl-1H-pyrazole-4-carboxamide, N-{2-[3-chloro-5-(trifluoromethyl)pyridin-2-yl]ethyl}-2-(trifluoromethyl)benzamide, natamycin, N-ethyl-N-methyl-N'-{2-methyl-5-(trifluoromethyl)-4-[3-(trimethylsilyl)propoxy]phenyl}imidoformamide, N-ethyl-N-methyl-N'-{2-methyl-5-(difluoromethyl)-4-[3-(trimethylsilyl)propoxy]phenyl}imidoformamide, nickel dimethyldithiocarbamate, nitrothal-isopropyl, O-{1-[(4-methoxyphenoxy)methyl]-2,2-dimethylpropyl}1H-imidazole-1-carbothioate, octhilinone, oxamocarb, oxyfenthiin, pentachlorophenol and salts, phosphorous acid and its salts, piperalin, propamocarb fosetylate, propanosine-sodium, proquinazid, pyribencarb, pyrrolnitrine, quintozene, S-allyl-5-amino-2-isopropyl-4-(2-methylphenyl)-3-oxo-2,3-dihydro-1H-pyrazole-1-carbothioate, tecloftalam, tecnazene, triazoxide, trichlamide, valiphenal, zarilamid.

Preferably, the fungicide compound (c) is selected in the list consisting of:
N-[2-(1,3-dimethylbutyl)phenyl]-5-fluoro-1,3-dimethyl-1H-pyrazole-4-carboxamide, benalaxyl, ethirimol, hymexazol, mefenoxam, metalaxyl, metalaxyl-M, benomyl, carbendazim, fuberidazole, pencycuron, thiabendazole, zoxamide, boscalid, carboxin, flutolanil, furametpyr, penthiopyrad, thifluzamide, azoxystrobin, cyazofamid, dimoxystrobin, famoxadone, fenamidone, fluoxastrobin, metominostrobin, orysastrobin, picoxystrobin, pyraclostrobin, trifloxystrobin, fluazinam, silthiofam, cyprodinil, kasugamycin, mepanipyrim, pyrimethanil, fenpiclonil, fludioxonil, iprodione, procymidone, propamocarb, tolclofos-methyl, bitertanol, cyproconazole, difenoconazole, diniconazole, epoxiconazole, etaconazole, fenhexamid, fluquinconazole, flutriafol, hexaconazole, imazalil, imibenconazole, ipconazole, metconazole, prochloraz, prothioconazole, simeconazole, spiroxamine, tebuconazole, tetraconazole, triadimefon, triadimenol, triflumizole, triticonazole, carpropamid, tolylfluanid, fluopicolide, isotianil, N-{2-[1,1'-bi(cyclopropyl)-2-yl]phenyl}-3-(difluoromethyl)-, 1-methyl-1H-pyrazole-4-carboxamide, propamocarb fosetylate, triazoxide, N-(3',4'-dichloro-5-fluorobiphenyl-2-yl)-3-(difluoromethyl)-1-methyl-1H-pyrazole-4-carboxamide, N-{2-[3-chloro-5-(trifluoromethyl)pyridin-2-yl]ethyl}-2-(trifluoromethyl)benzamide.

More preferably, the fungicide compound (c) is selected in the list L1 consisting of:
N-[2-(1,3-dimethylbutyl)phenyl]-5-fluoro-1,3-dimethyl-1H-pyrazole-4-carboxamide, metalaxyl, carbendazim, pencycuron, fenamidone, fluoxastrobin, trifloxystrobin, pyrimethanil, iprodione, bitertanol, fluquinconazole, ipconazole, prochloraz, prothioconazole, tebuconazole, triadimenol, triticonazole, carpropamid, tolylfluanid, fluopicolide, isotianil, N-{2-[1,1'-bi(cyclopropyl)-2-yl]phenyl}-3-(difluoromethyl)-, 1-methyl-1H-pyrazole-4-carboxamide, propamocarb fosetylate, N-(3',4'-dichloro-5-fluorobiphenyl-2-yl)-3-(difluoromethyl)-1-methyl-1H-pyrazole-4-carboxamide, N-{2-[3-chloro-5-(trifluoromethyl)pyridin-2-yl]ethyl}-2-(trifluoromethyl)benzamide, fludioxonil, mefenoxam, pyraclostrobin, boscalid, azoxystrobin.

Where the third active ingredient (c) as defined above is present in the composition, this compound may be present in an amount of (a) / (b) / (c) weight ratio of from 1 / 1 / 1 to 1 /$10^{14}$ / $10^{14}$; the ratios of compounds (a), (b) and (c) varying independently from each other. Preferably, (a) / (b) / (c) weight ratio is of from 1/10/10 to 1/$10^{13}$/$10^{13}$. Even more preferably, (a) / (b) / (c) weight ratio is of from 1/$10^2$/$10^2$ to 1/$10^{12}$/$10^{12}$, from 1/$10^2$/$10^2$ to 1/$10^8$/$10^8$, from 1/$10^3$/$10^3$ to 1/$10^6$/$10^6$ , from 1/$10^3$/$10^3$ to 1/$10^5$/$10^5$.

When the composition is applied via a seed treatment, the (a) / (b) / (c) weight ratio can be advantageously from 1/$10^2$/$10^2$ to 1/$10^8$/$10^8$, preferably from 1/$10^3$/$10^3$ to 1/$10^6$/$10^6$ , even more preferably from 1/$10^3$/$10^3$ to 1/$10^5$/$10^5$.

A man of ordinary skill in the art would be able to determine the adequate ratios according to the methods of application and to the compounds.

Non limitative examples of suitable mixtures according to the present invention may include mixtures of :

- compound (I) with a first fungicide compound selected in the list L and a second fungicide compound different from the first one selected from the list L1;

The composition according to the present invention may further comprise a supplementation with the AM fungi, which can be added as spores or other inoculum. Such AM fungi could be for example *Glomus sp., Gigaspora sp.,* or other fungi from the group Glomeromycota.

The composition according to the present invention may further comprise another additional component such as an agriculturally acceptable support, carrier or filler.

In the present specification, the term "support" denotes a natural or synthetic, organic or inorganic material with which the active material is combined to make it easier to apply, notably to the parts of the plant. This support is thus generally inert and should be agriculturally acceptable. The support may be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports may also be used.

The composition may also comprise other additional components. In particular, the composition may further comprise a surfactant. The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the above compounds containing sulphate, sulphonate and phosphate functions. The presence of at least one surfactant is generally essential when the active material and/or the inert support are water-insoluble and when the vector agent for the application is water. Preferably, surfactant content may be comprised between 5% and 40% by weight of the composition.

Additional components may also be included, e.g. protective colloids, adhesives, thickeners, thixotropic agents, penetration agents, stabilisers, sequestering agents. More generally, the active materials can be combined with any solid or liquid additive, which complies with the usual formulation techniques.

In general, the composition according to the invention may contain from 0.05 to 99% (by weight) of active material, preferably 10 to 70% by weight.

Compositions according to the present invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ulv) liquid, ultra low volume (ulv) suspension, water dispersible granules or tablets, water dispersible powder for slurry treatment, water soluble granules or tablets, water soluble powder for seed treatment and wettable powder.

These compositions include not only compositions which are ready to be applied to the plant or seed to be treated, or in furrow in the soil, by means of a suitable device, such as a spraying or dusting device, but also concentrated commercial compositions which must be diluted before they are applied to the crop.

Protocols to synthesise the compound of formula (I) according to the invention are well known by those skilled in the art (Bouwmeester et al, 2003, Current opinion in Plant Biology 6: 358-364; Mangnus et al., 1992, J. Agric. Food Chem. 40(6):697-700; Frischmuth et al, 1991, Tetrahedron 47:9793-9806; Mwakaboko A.S., 2003, "Synthesis and biological evaluation of new strigolactone analogues as germination stimulants for the seeds of the parasitic weeds Striga and Orobanche spp", Doctoral thesis, http://webdoc.ubn.kun.nl /mono/m/mwakaboko_a/syntanbie.pdf).
As an example, Mwakaboko A.S. discloses in his thesis the synthesis of some structurally modified strigolactones (chapter 8 & 9).
The pesticidal compositions of the present invention can be used to curatively or preventively control phytopathogenic fungi of crops, but also to increase the yield, growth, or vigor of the plant. Additionally, the present invention used preventively may control parasitic weed species.
Thus, according to a further aspect, the present invention provides a method for curatively or preventively controlling phytopathogenic fungi of crops and/or increasing the yield, growth or vigor of a plant characterised in that a composition according to the invention is applied via seed treatment, foliar application, stem application, drench/drip application (chemigation) to the seed, the plant or to the fruit of the plant, or to soil, particularly in furrow, and/or to inert substrate (e.g. inorganic substrates (e.g. sand, rockwool, glasswool, expanded minerals (e.g. perlite, vermiculite, zeolite, expanded clay)), Pumice, Pyroclastic materials/tuff, synthetic organic substrates (e.g. Polyurethane), organic substrates (e.g. peat, composts, tree waste products (e.g. coir, wood fibre/chips, tree bark)) and/or to a liquid substrate (e.g. floating hydroponic systems, Nutrient Film Technique, Aeroponics) wherein the plant is growing or wherein it is desired to grow.
The composition as used against phytopathogenic fungi comprises an effective and non-phytotoxic amount of a fungicide compound.
The expression "effective and non-phytotoxic amount" means an amount of composition according to the invention which is sufficient to control or destroy the pests and diseases present or liable to appear on the crops, and which does not entail any appreciable symptom of phytotoxicity for the said crops. Such an amount can vary within a wide range depending on the pests and diseases to be combated or controlled, the type of crop, the climatic conditions and the compounds included in the composition according to the invention.
This amount can be determined by systematic field trials, which are within the capabilities of a person skilled in the art.
The method of treatment according to the present invention is useful to treat propagation material such as tubers or rhizomes, but also seeds, seedlings or seedlings pricking out and plants or plants pricking out. This method of treatment can also be useful to treat roots. The method of treatment according to the present invention can also be useful to treat the overground parts of the plant such as trunks, stems or stalks, leaves, flowers and fruit of the concerned plant.
Among the plants that can be treated by the method according to the present invention, mention may be made of cotton; flax; vine; fruit or vegetable crops such as *Rosaceae sp.* (for instance pip fruit such as apples and pears, but also stone fruit such as apricots, almonds and peaches), *Ribesioidae sp.*, *Juglandaceae sp., Betulaceae sp., Anacardiaceae sp., Fagaceae sp., Moraceae sp.*, *Oleaceae sp., Actinidaceae sp., Lauraceae sp., Musaceae sp.* (for instance banana trees and plantins), *Rubiaceae sp.*, *Theaceae sp., Sterculiceae sp., Rutaceae sp.* (for instance lemons, oranges and grapefruit); *Solanaceae sp.* (for instance tomatoes), *Liliaceae sp.*, *Asteraceae sp.* (for instance lettuces), *Umbelliferae sp., Cruciferae sp., Chenopodiaceae sp., Cucurbitaceae sp.*, *Papilionaceae sp.* (for instance peas), *Rosaceae sp.* (for instance strawberries); major crops such as *Graminae sp.* (for instance maize, lawn or cereals such as wheat, rice, barley and triticale), *Asteraceae sp.* (for instance sunflower), *Cruciferae sp.* (for instance colza), *Fabacae sp.* (for instance peanuts), *Papilionaceae sp.* (for instance soybean), *Solanaceae sp.* (for instance potatoes), *Chenopodiaceae sp.* (for instance beetroots); horticultural and forest crops; as well as genetically modified homologues of these crops.
Among the diseases of plants or crops that can be controlled by the method according to the present invention, mention may be made of:

Powdery mildew diseases such as :

Blumeria diseases, caused for example by *Blumeria graminis*;
Podosphaera diseases, caused for example by *Podosphaera leucotricha*;
Sphaerotheca diseases, caused for example by *Sphaerotheca fuliginea*;
Uncinula diseases, caused for example by *Uncinula necator*;

Rust diseases such as :

Gymnosporangium diseases, caused for example by *Gymnosporangium sabinae;*
Hemileia diseases, caused for example by *Hemileia vastatrix*;
Phakopsora diseases, caused for example by *Phakopsora pachyrhizi* or *Phakopsora meibomiae*;

Puccinia diseases, caused for example by *Puccinia recondita*;
Uromyces diseases, caused for example by *Urmyces appendiculatus*;

Oomycete diseases such as :

Bremia diseases, caused for example by *Bremia lactucae*;
Peronospora diseases, caused for example by *Peronospora pisi* or *P. brassicae*;
Phytophthora diseases, caused for example by *Phytophthora infestans*;
Plasmopara diseases, caused for example by *Plasmopara viticola;*
Pseudoperonospora diseases, caused for example by *Pseudoperonospora humuli* or *Pseudoperonospora cubensis*;
Pythium diseases, caused for example by *Pythium ultimum*;

Leafspot, leaf blotch and leaf blight diseases such as :

Alternaria diseases, caused for example by *Alternaria solani*;
Cercospora diseases, caused for example by *Cercospora beticola*;
Cladiosporum diseases, caused for example by *Cladiosporium cucumerinum*;
Cochliobolus diseases, caused for example by *Cochliobolus sativus*;
Colletotrichum diseases, caused for example by *Colletotrichum lindemuthanium*;
Cycloconium diseases, caused for example by *Cycloconium oleaginum*;
Diaporthe diseases, caused for example by *Diaporthe citri*;
Elsinoe diseases, caused for example by *Elsinoe fawcettii*;
Gloeosporium diseases, caused for example by *Gloeosporium laeticolor*;
Glomerella diseases, caused for example by *Glomerella cingulata*;
Guignardia diseases, caused for example by *Guignardia bidwelli*;
Leptosphaeria diseases, caused for example by *Leptosphaeria maculans*; *Leptosphaeria nodorum*;
Magnaporthe diseases, caused for example by *Magnaporthe grisea*;
Mycosphaerella diseases, caused for example by *Mycosphaerella graminicola*; *Mycosphaerella arachidicola*; *Mycosphaerella fijiensis*;
Phaeosphaeria diseases, caused for example by *Phaeosphaeria nodorum*;
Pyrenophora diseases, caused for example by *Pyrenophora teres*;
Ramularia diseases, caused for example by *Ramularia collo-cygni*;
Rhynchosporium diseases, caused for example by *Rhynchospoium secalis*;
Septoria diseases, caused for example by *Septoria apii* or *Septoria lycopercisi*;
Typhula diseases, caused for example by *Typhula incarnata*;
Venturia diseases, caused for example by *Venturia inaequalis*;

Root and stem diseases such as :

Corticium diseases, caused for example by *Corticium graminearum*;
Fusarium diseases, caused for example by *Fusarium oxysporum*;
Gaeumannomyces diseases, caused for example by *Gaeumannomyces graminis*;
Rhizoctonia diseases, caused for example by *Rhizoctonia solani*;
Tapesia diseases, caused for example by *Tapesia acuformis*;
Thielaviopsis diseases, caused for example by *Thielaviopsis basicola*;

Ear and panicle diseases such as :

Alternaria diseases, caused for example by *Alternaria spp*.;
Aspergillus diseases, caused for example by *Aspergillus flavus*;
Cladosporium diseases, caused for example by *Cladosporium spp*.;
Claviceps diseases, caused for example by *Claviceps purpurea*;
Fusarium diseases, caused for example by *Fusarium culmorum*;
Gibberella diseases, caused for example by *Gibberella zeae*;
Monographella diseases, caused for example by *Monographella nivalis*;

Smut and bunt diseases such as :

Sphacelotheca diseases, caused for example by *Sphacelotheca reiliana*;
Tilletia diseases, caused for example by *Tilletia caries*;
Urocystis diseases, caused for example by *Urocystis occulta*;
Ustilago diseases, caused for example by *Ustilago nuda*;

Fruit rot and mould diseases such as :

Aspergillus diseases, caused for example by *Aspergillus flavus*;
Botrytis diseases, caused for example by *Botrytis cinerea*;
Penicillium diseases, caused for example by *Penicillium expansum*;
Sclerotinia diseases, caused for example by *Sclerotinia sclerotiorum*;
Verticilium diseases, caused for example by *Verticilium alboatrum*;

Seed and soilborne decay, mould, wilt, rot and damping-off diseases :

Fusarium diseases, caused for example by *Fusarium culmorum*;
Phytophthora diseases, caused for example by *Phytophthora cactorum*;
Pythium diseases, caused for example by *Pythium ultimum*;
Rhizoctonia diseases, caused for example by *Rhizoctonia solani*;
Sclerotium diseases, caused for example by *Sclerotium rolfsii*;
Microdochium diseases, caused for example by *Microdochium nivale*;

Canker, broom and dieback diseases such as :

Nectria diseases, caused for example by *Nectria galligena*;

Blight diseases such as :

Monilinia diseases, caused for example by *Monilinia laxa*;

Leaf blister or leaf curl diseases such as :

Taphrina diseases, caused for example by *Taphrina deformans*;

Decline diseases of wooden plants such as :

Esca diseases, caused for example by *Phaemoniella clamydospora*;

Diseases of flowers and Seeds such as :

Botrytis diseases, caused for example by *Botrytis cinerea;*

Diseases of tubers such as:

Rhizoctonia diseases, caused for example by *Rhizoctonia solani*.

The fungicide composition according to the present invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds of the present invention, or a composition according to the invention; this includes for example direct application, spraying, dipping, injection or any other suitable means.

The composition according to the present invention may also be used in the treatment of genetically modified organisms with the compounds according to the invention or the agrochemical compositions according to the invention. Genetically modified plants are plants into whose genome a heterologous gene encoding a protein of interest has been stably integrated. The expression "heterologous gene encoding a protein of interest" essentially means genes which give the transformed plant new agronomic properties, or genes for improving the agronomic quality of the transformed plant.

The dose of active material usually applied in the treatment according to the present invention is generally and advantageously between 10 and 800 g/ha, preferably between 50 and 300 g/ha for applications in foliar treatment. If a drench/drip/in furrow application is possible, the dose can be lower, especially in artificial substrates like rockwool or perlite. The dose of active substance applied is generally and advantageously between 0.5 and 200 g per 100 kg of seed, preferably between 1 and 150 g per 100 kg of seed in the case of seed treatment. It is clearly understood that the doses indicated above are given as illustrative examples of the invention. A person skilled in the art will know how to adapt the application doses according to the nature of the crop to be treated.

**Biological tests:**

**1. Calculation of the fungicide synergistic effect**

A synergistic effect of fungicides is always present when the fungicidal activity of the active compound combinations exceeds the total of the activities of the active compounds when applied individually.
The expected activity for a given combination of two active compounds can be calculated as follows (cf. Colby, S.R., "Calculating Synergistic and Antagonistic Responses of Herbicide Combinations", Weeds 15, pages 20-22, 1967):
If

X is the efficacy, when applying the active compound A at a rate of application of active compound of m g/ha,
Y is the efficacy, when applying the active compound B at a rate of application of active compound of n g/ha,
E is the expected efficacy, when applying the active compounds A and B at rates of application of active compound of m and n g/ha,

then

$$E = X + Y - \frac{X \cdot Y}{100}$$

The degree of efficacy, expressed in % is denoted. 0% means an efficacy which corresponds to that of the control while an efficacy of 100% means that no disease is observed.
If the actual fungicidal activity exceeds the calculated value, then the activity of the combination is superadditive, i.e. a synergistic effect exists. In this case, the efficacy which was actually observed must be greater than the value for the expected efficacy (E) calculated from the abovementioned formula.

**2. *Botrytis cinerea* - test**

The microtest was performed in liquid medium with potato-dextrose broth (PDB) using microtitre plates.
The active compound is applied as the technical active substance dissolved in methanol.
A spore suspension of *Botrytis cinerea* was used for inoculation. After 5 days of incubation by darkness under shaking (10 Hrz), the optical density in each cavity was evaluated with the aid of a microtitre plate reader.
0% means an efficacy which corresponds to that of the control, while an efficacy of 100% means that no fungal growth is observed.
The tables below clearly show that the observed activities of the active compound combinations according to the invention are greater than the calculated activity, i.e. a synergistic effect is present.

| Active compound<br>Known: | Rate of application of active compound in ppm | Efficacy in % |
|---|---|---|
| N-[2-(1,3-Dimethylbutyl)phenyl]-5-fluor-1,3-dimethyl-1H-pyrazol-4-carboxamid | 0.001 | 17 |

(continued)

| Active compound<br>Known: | Rate of application of active compound in ppm | Efficacy in % |
|---|---|---|
| 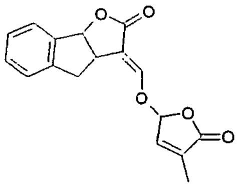<br>Compound A | 0.0000001 | 8 |

**Inventive Compound combination:**

| | Ratio of the mixture | Rate of application of active compound in ppm | Actual Efficacy (%) | Expected value, calculated using Colby's formula |
|---|---|---|---|---|
| N-[2-(1,3-Dimethylbutyl)phenyl]-5-fluor-1,3-dimethyl-1H-pyrazol-4-carboxamid + Compound A } | **10000:1** | 0.001 + 0.0000001 } | **29** | **24** |

| Active compound<br>Known: | Rate of application of active compound in ppm | Efficacy in % |
|---|---|---|
| Prothioconazole | 0.3 | 40 |
| Compound A<br>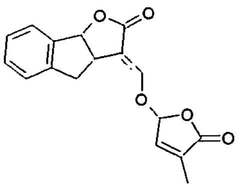 | 0.00003 | 1 |

**Inventive Compound combination:**

| | Ratio of the mixture | Rate of application of active compound in ppm | Actual Efficacy (%) | Expected value, calculated using Colby's formula |
|---|---|---|---|---|
| Prothioconazole + Compound A } | **10000:1** | 0.3 + 0.00003 } | **52** | **41** |

***3. Rhizoctonia solani*** **test**

The microtest was performed in liquid medium with potato-dextrose broth (PDB) using microtitre plates.
The active compound is applied as the technical active substance dissolved in methanol.
A mycelium suspension of *Rhizoctonia solani* was used for inoculation. After 5 days of incubation by darkness under shaking (10 Hrz), the optical density in each cavity was evaluated with the aid of a microtitre plate reader.
0% means an efficacy which corresponds to that of the control, while an efficacy of 100% means that no fungal growth is observed.
The table below clearly shows that the observed activity of the active compound combination according to the invention is greater than the calculated activity, i.e. a synergistic effect is present.

| Active compound Known: | Rate of application of active compound in ppm | Efficacy in % |
|---|---|---|
| Metalaxyl | 0.001 | 27 |
| Compound A 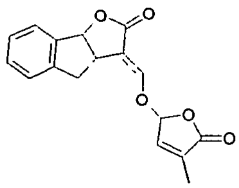 | 0.0000001 | 26 |

**Inventive Compound combination:**

| | Ratio of the mixture | Rate of application of active compound in ppm | Actual Efficacy (%) | Expected value, calculated using Colby's formula |
|---|---|---|---|---|
| Metalaxyl + Compound A | **10000:1** | 0.001 + 0.0000001 | **72** | **46** |

***4. Gibberella zeae*** **test**

The microtest was performed in liquid medium with potato-dextrose broth (PDB) using microtitre plates.
The active compound is applied as the technical active substance dissolved in methanol.
A spore suspension of *Gibberella zeae* was used for inoculation. After 3 days of incubation by darkness under shaking (10 Hrz), the optical density in each cavity was evaluated with the aid of a microtitre plate reader.
0% means an efficacy which corresponds to that of the control, while an efficacy of 100% means that no fungal growth is observed.
The table below clearly shows that the observed activity of the active compound combination according to the invention is greater than the calculated activity, i.e. a synergistic effect is present.

| Active compound Known: | Rate of application of active compound in ppm | Efficacy in % |
|---|---|---|
| Tebuconazole | 0.001 | 23 |
| Compound A | 0.0000001 | 35 |

(continued)

| Active compound Known: | Rate of application of active compound in ppm | Efficacy in % |
|---|---|---|
| 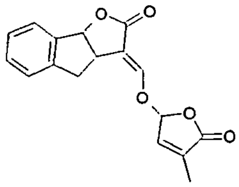 | | |

**Inventive Compound combination**:

| | Ratio of the mixture | Rate of application of active compound in ppm | Actual Efficacy (%) | Expected value, calculated using Colby's formula |
|---|---|---|---|---|
| Tebuconazole + Compound A | **10000:1** | 0.001 + 0.0000001 | **65** | **50** |

***5. Pyricularia oryzae* test:**

The microtest was performed in liquid medium with potato-dextrose broth (PDB) using microtitre plates.

The active compound is applied as the technical active substance dissolved in methanol.

A spore suspension of *Pyricularia oryzae* was used for inoculation. After 3 days of incubation by darkness under shaking (10 Hrz), the optical density in each cavity was evaluated with the aid of a microtitre plate reader.

0% means an efficacy which corresponds to that of the control, while an efficacy of 100% means that no fungal growth is observed.

The table below clearly shows that the observed activity of the active compound combination according to the invention is greater than the calculated activity, i.e. a synergistic effect is present.

| Active compound Known: | Rate of application of active compound in ppm | Efficacy in % |
|---|---|---|
| Trifloxystrobin | 0.3 | 58 |
| Compound A 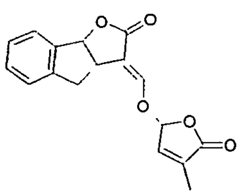 | 0.00003 | 4 |

**Inventive Compound combination:**

| | Ratio of the mixture | Rate of application of active compound in ppm | Actual Efficacy (%) | Expected value, calculated using Colby's formula |
|---|---|---|---|---|
| Trifloxystrobin + Compound A | **10000:1** | 0:3 + 0:00003 | **97** | **60** |

## Claims

1. A composition comprising:

   a) a strigolactone derivative of formula:

   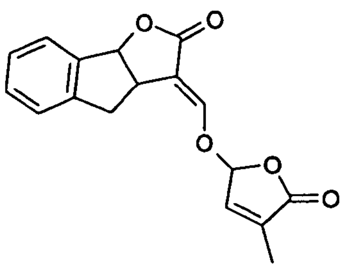

   and

   b) a fungicide compound selected in the list consisting of N-[2-(1,3-dimethylbutyl)phenyl]-5-fluoro-1,3-dimethyl-1H-pyrazole-4-carboxamide, metalaxyl, metalaxyl-M, fluoxastrobin, trifloxystrobin, prothioconazole, tebuconazole, triadimenol; in a (a)/(b) weight ratio of from 1/1 to $1/10^{14}$.

2. A composition according to any one of the claim 1 further comprising a fungicidal compound (c).

3. A composition according to claim 2, **characterised in that** the fungicidal compound (c) is selected from N-[2-(1,3-dimethylbutyl)phenyl]-5-fluoro-1,3-dimethyl-1H-pyrazole-4-carboxamide, metalaxyl, carbendazim, pencycuron, fenamidone, fluoxastrobin, trifloxystrobin, pyrimethanil, iprodione, bitertanol, fluquinconazole, ipconazole, prochloraz, prothioconazole, tebuconazole, triadimenol, triticonazole, carpropamid, tolylfluanid, fluopicolide, isotianil, N-{2-[1,1'-bi(cyclopropyl)-2-yl]phenyl}-3-(difluoromethyl)-, 1-methyl-1H-pyrazole-4-carboxamide, propamocarb fosetylate, N-(3',4'-dichloro-5-fluorobiphenyl-2-yl)-3-(difluoromethyl)-1-methyl-1H-pyrazole-4-carboxamide, N-{2-[3-chloro-5-(trifluoromethyl)pyridin-2-yl]ethyl}-2-(trifluoromethyl)benzamide, fludioxonil, mefenoxam, pyraclostrobin, boscalid, azoxystrobin.

4. A composition according to any one of the claims 1 to 3, **characterised in that** it further comprises an agriculturally acceptable support, carrier, filler and/or surfactant.

5. A composition according to any one of the claims 1 to 4, **characterized in that** it further comprises a supplementation with Arbuscular mycorrhizae fungi.

6. A method for curatively or preventively controlling phytopathogenic fungi of crops **characterised in that** an effective and non-phytotoxic amount of a composition according to any one of the claims 1 to 5 is applied via seed treatment, foliar application, stem application, drench/drip application (chemigation) to the seed, the plant or to the fruit of the plant or to soil or to inert substrate, Pumice, Pyroclastic materials/tuff, synthetic organic substrates, organic substrates or to a liquid substrate in which the plant is growing or in which it is desired to grow.

7. A method according to claim 6, **characterized in that** the composition is applied in furrow on the soil

8. The use of the composition as claimed in any one of the claims 1 to 5 for curatively or preventively controlling phytopathogenic fungi.

9. The use of the composition as claimed in any one of the claims 1 to 5 for preventively controlling phytopathogenic fungi and parasitic weed species.

## Patentansprüche

1. Zusammensetzung, die Folgendes umfasst:

   a) ein Strigolactonderivat der Formel:

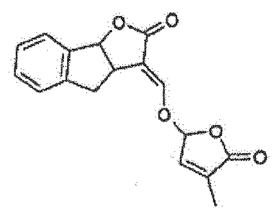

   und
   b) eine Fungizidverbindung, ausgewählt aus der Reihe N-[2-(1,3-Dimethylbutyl)phenyl]-5-fluor-1,3-dimethyl-1H-pyrazol-4-carboxamid, Metalaxyl, Metalaxyl-M, Fluoxastrobin, Trifloxystrobin, Prothioconazol, Tebuconazol, Triadimenol; in einem Gewichtsverhältnis (a)/(b) von 1/1 bis $1/10^{14}$.

2. Zusammensetzung nach Anspruch 1, die weiterhin eine fungizide Verbindung (c) umfasst.

3. Zusammensetzung nach Anspruch 2, **dadurch gekennzeichnet, dass** die fungizide Verbindung (c) aus der folgenden Reihe ausgewählt ist: N-[2-(1,3-Dimethylbutyl)phenyl]-5-fluor-1,3-dimethyl-1H-pyrazol-4-carboxamid, Metalaxyl, Carbendazim, Pencycuron, Fenamidon, Fluoxastrobin, Trifloxystrobin, Pyrimethanil, Iprodin, Bitertanol, Fluquinoconazol, Ipconazol, Prochloraz, Prothioconazol, Tebuconazol, Triadimenol, Triticonazol, Carpropamid, Tolylfluanid, Fluopicolid, Isotianil, N-{2-[1,1'-Bi(cyclopropyl)-2-yl]phenyl}-3-(difluormethyl)-, 1-Methyl-1H-pyrazol-4-carboxamid, Propamocarbfosetylat, N-(3',4'-Dichlor-5-fluorbiphenyl-2-yl)-3-(difluormethyl)-1-methyl-1H-pyrazol-4-carboxamid, N-{2-[3-Chlor-5-(trifluormethyl)pyridin-2-yl]ethyl}-2-(trifluormethyl)benzamid, Fludioxonil, Mefenoxam, Pyraclostrobin, Boscalid, Azoxystrobin.

4. Zusammensetzung nach einem der Ansprüche 1 bis 3, **dadurch gekennzeichnet, dass** sie weiterhin einen landwirtschaftlich unbedenklichen Träger, Grundstoff, Füllstoff und/oder ein landwirtschaftlich unbedenkliches Tensid umfasst.

5. Zusammensetzung nach einem der Ansprüche 1 bis 4, **dadurch gekennzeichnet, dass** sie weiterhin eine Ergänzung mit Arbuskulären Mykorrhiza-Pilzen umfasst.

6. Verfahren für die kurative oder präventive Bekämpfung von phytopathogenen Pilzen an Kulturen, **dadurch gekennzeichnet, dass** man eine wirksame und nichtphytotoxische Menge einer Zusammensetzung nach einem der Ansprüche 1 bis 5 durch Saatgutbehandlung, Blattapplikation, Stängelapplikation, Tränk/Tröpfchenapplication (Chemigation) auf den Samen, die Pflanze oder die Frucht der Pflanze oder auf Boden oder auf inertes Substrat, Bimsstein, pyroklastisches Material/Tuff, synthetische organische Substrate, organische Substrate oder auf ein flüssiges Substrat, in dem die Pflanze wächst oder wachsen soll, ausbringt.

7. Verfahren nach Anspruch 6, **dadurch gekennzeichnet, dass** die Zusammensetzung in die Furche auf den Boden ausgebracht wird.

8. Verwendung der Zusammensetzung nach einem der Ansprüche 1 bis 5 für die kurative oder präventive Bekämpfung von phytopathogenen Pilzen.

**9.** Verwendung der Zusammensetzung nach einem der Ansprüche 1 bis 5 für die präventive Bekämpfung von phytopathogenen Pilzen und parasitischen Unkrautarten.

## Revendications

**1.** Composition comprenant :

a) un dérivé de strigolactones de formule

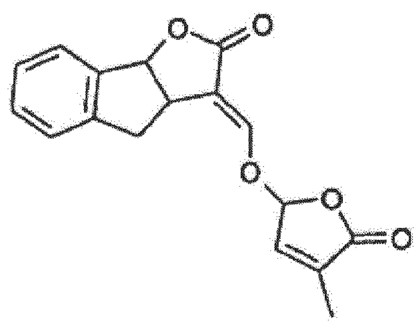

et

b) un composé fongicide choisi dans la liste constituée par le N-[2-(1,3-diméthylbutyl)phényl]-5-fluoro-1,3-diméthyl-1H-pyrazole-4-carboxamide, le métalaxyle, le métalaxyle-M, la fluoxastrobine, la trifloxystrobine, le prothioconazole, le tébuconazole, le triadiménol ;

selon un rapport pondéral (a)/(b) allant de 1/1 à $1/10^{14}$.

**2.** Composition selon la revendication 1, comprenant en outre un composé fongicide (c).

**3.** Composition selon la revendication 2, **caractérisée en ce que en ce que** le composé fongicide (c) est choisi parmi le N-[2-(1,3-diméthylbutyl)phényl]-5-fluoro-1,3-diméthyl-1H-pyrazole-4-carboxamide, le métalaxyle, le carbendazime, le pencycuron, la fénamidone, la fluoxastrobine, la trifloxystrobine, le pyriméthanil, l'iprodione, le bitertanol, le fluquinconazole, l'ipconazole, le prochloraz, le prothioconazole, le tébuconazole, le triadiménol, le triticonazole, le carpropamide, le tolylfluanide, le fluopicolide, l'isotianil, le N-{2-[1,1'-bi(cyclopropyl)-2-yl]phényl}-3-(difluorométhyl)-1-méthyl-1H-pyrazole-4-carboxamide, le propamocarb fosétylate, le N-(3',4'-dichloro-5-fluorobiphényl-2-yl)-3-(difluorométhyl)-1-méthyl-1H-pyrazole-4-carboxamide,leN-{2-[3-chloro-5-(trifluorométhyl)pyridin-2-yl]éthyl}-2-(trifluorométhyl)benzamide, le fludioxonil, le méfénoxam, la pyraclostrobine, le boscalide, l'azoxystrobine.

**4.** Composition selon l'une quelconque des revendications 1 à 3, **caractérisée en ce qu'**elle comprend en outre un support, un véhicule, une charge et/ou un agent tensioactif acceptable sur le plan agricole.

**5.** Composition selon l'une quelconque des revendications 1 à 4, **caractérisée en ce qu'**elle comprend en outre une complémentation par le champignon *Arbuscular mycorrhizae.*

**6.** Méthode de contrôle préventif ou curatif de champignons phytopathogènes sur des cultures, **caractérisée en ce qu'**une quantité efficace et non phytotoxique d'une composition selon l'une quelconque des revendications 1 à 5 est appliquée par l'intermédiaire d'un traitement des semences, d'une application foliaire, d'une application aux tiges, d'une application par goutte à goutte/pulvérisation (application par irritation) aux semences, à la plante ou aux fruits de la plante ou au sol ou à un substrat inerte, des matériaux de pierre ponce, pyroclastiques/tuf volcanique, des substrats organiques synthétiques, des substrats organiques ou à un substrat liquide dans lequel la plante se développe ou dans lequel on souhaite qu'elle se développe.

**7.** Méthode selon la revendication 6, **caractérisée en ce que** la composition est appliquée en sillon dans le sol.

**8.** Utilisation de la composition selon l'une quelconque des revendications 1 à 5, pour le contrôlé préventif ou curatif de champignons phytopathogènes.

**9.** Utilisation de la composition selon l'une quelconque des revendications 1 à 5, pour le contrôle préventif de champignons phytopathogènes et d'espèces d'adventices parasites.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005077177 A

### Non-patent literature cited in the description


- **COOK et al.** *J Am Chem Soc,* 1972, vol. 94, 6198-6199
- **BUTLER.** *Allelopathy: organism, processes and application,* 1995, 158-168
- **HAUCK et al.** *J Plant Physiol,* 1992, vol. 139, 474-478
- **MULLER et al.** *J Plant Growth Regul,* 1992, vol. 11, 77-84
- **SIAME et al.** *J Agr Food Chem,* 1993, vol. 41, 1486-1491
- **YOKOTA et al.** *Phytochemistry,* 1998, vol. 49, 1967-1973
- **AKIYAMA K. ; H. HAYASHI H.** Annals of Botany. 2006, vol. 97, 925-931
- Calculation of the synergistic and antagonistic responses of herbicide combinations. *Weeds,* 1967, vol. 15, 20-22
- **BOUWMEESTER et al.** *Current opinion in Plant Biology,* 2003, vol. 6, 358-364
- **MANGNUS et al.** *J. Agric. Food Chem.,* 1992, vol. 40 (6), 697-700
- **FRISCHMUTH et al.** *Tetrahedron,* 1991, vol. 47, 9793-9806
- **MWAKABOKO A.S.** Synthesis and biological evaluation of new strigolactone analogues as germination stimulants for the seeds of the parasitic weeds Striga and Orobanche spp. *Doctoral thesis,* 2003, http://webdoc.ubn.kun.nl /mono/m/mwakaboko_a/syntanbie.pdf
- **COLBY, S.R.** Calculating Synergistic and Antagonistic Responses of Herbicide Combinations. *Weeds,* 1967, vol. 15, 20-22